(19) Europäisches Patentamt
European Patent Office
Office européen des brevets

(11) Publication number: 0 352 800 A2

(12) **EUROPEAN PATENT APPLICATION**

(21) Application number: 89113903.2

(22) Date of filing: 27.07.89

(51) Int. Cl.[4]: C08J 3/07 , A61K 9/30 , //(C08L1/08,33:12)

(30) Priority: 28.07.88 US 225458

(43) Date of publication of application: 31.01.90 Bulletin 90/05

(84) Designated Contracting States: AT BE CH DE ES FR GB GR IT LI LU NL SE

(71) Applicant: WARNER-LAMBERT COMPANY
201 Tabor Road
Morris Plains New Jersey 07950(US)

(72) Inventor: Ghebre-Sellassie, Isaac
359 Dell Place
Stanhope New Jersey 07874(US)
Inventor: Huang, Hua-Pin
24 Horizon Drive
Succasunna New Jersey 07876(US)

(74) Representative: Mansmann, Ivo
c/o Gödecke AG - Patentabteilung Postfach
569 - Mooswaldallee 1-9
D-7800 Freiburg(DE)

(54) **Aqueous polymeric dispersion of cellulosic and acrylic based polymers for preparing pharmaceutical dosage forms and dosage forms thereof.**

(57) The present invention is a novel dispersion process of preparing the dispersion or coating therefrom for use in preparing dosage forms of a pharmaceutically active ingredient. The process is mixing (preferably dissolving) a cellulose-derived polymer, such as cellulose ether, and an acrylic based polymer, such as a polyacrylate in an organic solvent, preferably methylene chloride, and then emulsifying the mixture in water followed by removing the organic solvent. The invention is also granules, tablets capsules, and emulsions prepared from the dispersion for use in pharmaceutical compositions.

EP 0 352 800 A2

# AQUEOUS POLYMERIC DISPERSION OF CELLULOSIC AND ACRYLIC BASED POLYMERS FOR PREPARING PHARMACEUTICAL DOSAGE FORMS AND DOSAGE FORMS THEREOF

## BACKGROUND OF THE INVENTION

The present invention relates to improved coating systems and a solid dosage form containing pharmaceutically active substances prepared using the improved coating systems, particularly a modified-release solid dosage form.

Coating systems of either organic or aqueous solvent systems for use in making pharmaceutical products are well known. Increasing concerns for environmental contamination, safety, and the expense associated with the use of organic solvents for selected polymers in coating systems made aqueous polymeric dispersions common for pharmaceutical use. However, each of these coating systems include drawbacks unique to each polymer of either the organic or aqueous solvent systems.

For example, the cellulose derived polymers using aqueous solvents require high curing temperatures while the acrylic polymers in organic solvents are tacky and require the addition of antitackiness agents in the final product.

Efforts to overcome these problems resulted in the use of mixtures of polymers in coating a pharmaceutically active substance such as are described in British Patents No. 2,086,725 and No. 2,157,170. These mixtures are prepared by combining an aqueous dispersion of acrylic polymer such as ethyl acrylate methyl methacrylate having a molecular weight of 800,000 sold by Röhm Pharma GMBH, Darmstadt (Fed. Rep. of Germany) under the name of Eudragit® E30D, and an aqueous dispersion of a polymer derived from cellulose such as Aquacoat ® ECD-30 sold by FMC Corporation, Philadelphia (Pennsylvania, U.S.A.) That is, Aquacoat® ECD-30 and Eudragit® E30D are mixed to form a dispersion suitable for coatings. However, since this mixture is composed of two kinds of particles of different compositions, the degree of interaction during film formation is not as high as is desireable. On the other hand, the present invention's coating system in which the dispersion is prepared using an emulsion/solvent evaporation technique described below provide an improved coating system for pharmaceutical dosage forms and provide novel dosage forms thereof.

Other references showing mixtures of polymers related to the present invention are well known under European Applications No. 148,811 and No. 142,877, and Belgian Patent No. 901,328. However, such mixtures do not provide a microparticle having both disclosed polymers therein as found in the present invention. Particularly, the preparation of the particles shows the present invention is novel and unobvious.

That is, it is now found that dissolution or dispersion, preferably dissolution of two polymers; one swellable in water, i.e., acrylic-based polymer, and the second insoluble in water, i.e., cellulose-derived, which dissolution is in a single organic solvent, preferably methylene chloride, then formation of another dispersion in water, provides submicron microparticles; approximately nanoparticles (pseudolatex), having a novel composition wherein each nanoparticle is essentially the same composition. Such a composition provides unexpected cohesion desirable for use in pharmaceutical dosage forms.

## SUMMARY OF THE INVENTION

The present invention is

1) a process for the preparation of a dispersion which comprises 1) mixing a cellulose-derived polymer and an acrylic-based polymer in an organic solvent, preferably methylene chloride; 2) emulsifying the mixture of step 1) in water, and 3) removing the organic solvent from the emulsion;

2) The dispersion prepared in 1) above;

3) A composition prepared by essentially removing the water from the dispersion of 2) which is a pseudolatex having 15% to 30% weight per volume of solids (polymer mixture) in water;

4) A composition consisting of particles consisting essentially of a cellulose-derived polymer and an acrylic-based polymer;

5) A pharmaceutical dosage form having a coating consisting essentially of the composition of 4);

6) The dosage form of 5) which is a pellet, a granule, tablet, capsule, or suspension.

7) The process of preparing a pharmaceutical dosage form using the compositions of 3) or 4) for coating a solid pharmaceutical containing at least one pharmaceutically active compound and usual

adjuvants.

## DETAILED DESCRIPTION OF THE INVENTION

By the term "cellulose-derived polymer" in this invention is meant various water insoluble cellulose ethers and esters which are generally well known for extended-release dosage form formulation.

Thus, the basic structure of cellulose-derived polymers is

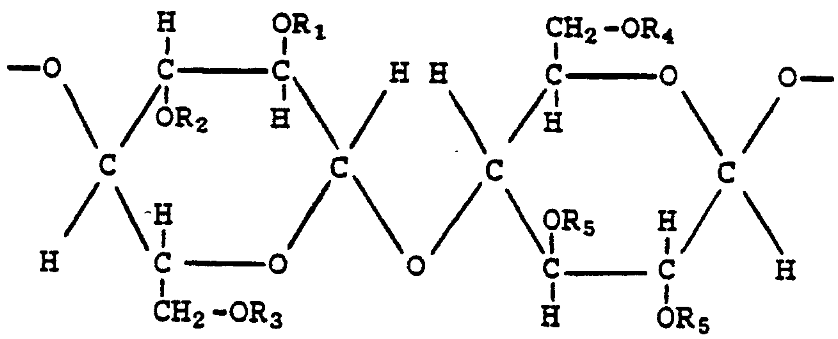

Formula 1

and may include the following

| | $R_1$ | $R_2$ | $R_3$ | $R_4$ | $R_5$ | $R_6$ |
|---|---|---|---|---|---|---|
| Cellulose acetate triacetate or tetraacetate | $-CO-CH_3$ | $-CO-CH_3$ | $-H$ | $-H$ | $-CO-CH_3$ | $-(CO-CH_3)$ |
| Cellulose acetate butyrate | $-CO-CH_3$ | $-CO-(CH_2)_2-CH_3$ | $-H$ | $-H$ | $-CO-CH_3$ | $-CO(CH_2)_2CH_3$ |
| Cellulose acetate phthalate | $-CO-CH_3$ | 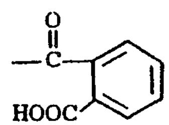 | 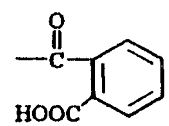 | 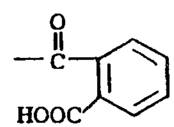 | $-CO-CH_3$ | 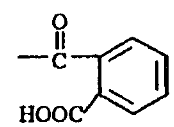 |
| Ethyl cellulose | $-CH_2-CH_3$ | $-H$ | $-CH_3-CH_3$ | $-CH_2-CH_3$ | $-H$ | $-CH_2-CH_3$ |

NOTE: Radicals R position from Formula 1 above.

The preferred cellulose-derived polymers in the present invention are cellulose ethers well known for formulation of extended release pharmaceutical dosage forms, for example, ethylcellulose (EC) and cellulose esters, that are further exemplified by cellulose acetate, cellulose acetate phthalate, and the like. Of these, the most preferred is ethyl cellulose.

By the term "acrylic-based polymer" in this invention is meant water swellable acrylic resins, also generally well known for formulation of extended-release pharmaceutical dosage forms. Particularly well known of these acrylic resins are the various Eudragit® resins as available from R ō hm Pharma GMBH shown as follows:

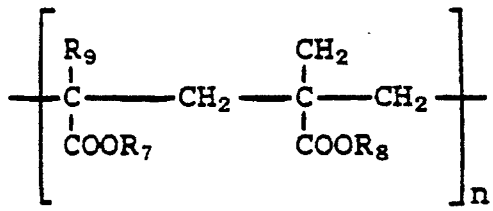

| Type | $R_7$ | $R_8$ | $R_9$ |
|---|---|---|---|
| Eudragit L | -H (50%) | $-CH_3$ | $-CH_3$ |
| Eudragit S | -H (30%) | $-CH_3$ | $-CH_3$ |
| Eudragit E | 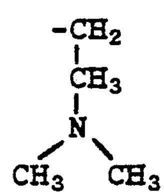 | $-CH_3$ $-C_4H_9$ | $-CH_3$ |
| Eudragit RL | $-CH_3$ $-CH_2H_3$ | 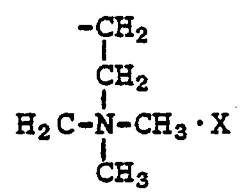 (10%) | -H $-CH_3$ |
| Eudragit RS | $-CH_3$ $-C_2H_5$ | 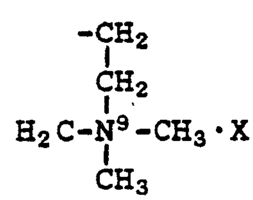 (5%) | -H $-CH_3$ |

The most preferred of the "acrylic-based polymers" for use in the present invention is Eudragit® RS.

That is, particularly preferred herein is Eudragit RS (RS) or RL.

In the present invention, the cellulose-derived polymer and the acrylic-based polymer are in the mixture in the weight ratio of 3:1 to 1:3, preferably in the ratio of 1:1 to 2:1.

The mixture of cellulose-derived polymer and acrylic-based polymer may or may not be in solution in the organic solvent. Thus, although a solution of the mixture is a preferred embodiment of the present invention, the mixture may be a dispersion such as is understood in the art to be colloids.

Generally, when solutions of two different polymers in a common solvent are mixed, they can undergo various interactions and lead to phase separation at polymer concentrations above a certain level. Incompatibility is the rule rather than the exception. However, surprisingly, in the preferred embodiment of the present invention, a clear solution is obtained when the preferred mixture of ethyl-cellulose or EC polymer and RS polymer, i.e., and Eudragit® RS-100, are dissolved in methylene chloride, indicating compatability of the polymers in the common solvent according to the present invention. Where the mixture is not a clear solution it is treated in the same manner as described hereinafter for a clear solution.

The clear solution is dispersed in water (EC/RS dispersion). Appropriate emulsion stabilizer may be added to the dispersion from those known in the art. Various surfactants with different chemical types may be used to stabilize the primary emulsion. Surfactants which may be used included polysorbates, Sorbitan fatty acid esters, and block copolymers of propylene oxide and ethylene oxide mixtures thereof. Fatty alcohols may be also used in conjunction with the above surfactants. A mixture of Polysorbate 80 (ICI Americas) and cetyl alcohol is the preferred surfactant for use in preparing our emulsion. The dispersion may then also be sonicated to form a primary organic solvent in water emulsion.

This primary emulsion may be passed through a microfluidizer to reduce the size of the droplets further and to obtain a stable final emulsion. No phase separation of the final emulsion is observed visually, after storage at room temperature for at least up to two months. In the primary emulsion, solvent droplets are not monodispersed and small droplets tended to coalesce with larger droplets. The larger the droplet size, the greater the tendency to coalesce, further increasing the droplet size. Therefore, microfluidization may be applied to the emulsion so the final emulsion contains monodispersed submicron droplets that do not coalesce and settle due to Brownian movement. A rough estimation of the droplet sizes is obtained from the appearance of the thin film formed on the container wall after draining. A blue to gray semitransparent film indicates that the droplet size is in the submicron range.

The dispersion or emulsion is then heated to from 38° C to 42° C, preferably to about 42° C under one atmosphere with stirring to drive off the organic solvent. After the removal of organic solvent, the evaporation process continues until a concentration of about 15% weight per volume polymer mixture in water is obtained. The evaporation process can be conducted by any means which would be known to those in the art, for example, at lower temperatures using a vacuum. The system of the present invention is a pseudolatex system having very fine particles dispersed in water. The light scattering property of the dispersion is similar to that described for the emulsion. The particle size is in the low submicron range. The dispersion is stable for at least two months. No sedimentation or agglomeration occurs in this period.

The organic solvent is taken off, for example, by evaporation, leaving the cellulose-derived polymer, such as EC, and acrylic-based polymer, such as RS, in each nanosphere. These nanospheres are the present invention. After solvent evaporation the preferred polymethacrylate, for example, Eudragit® RS is immobilized in the small particles and its quarternary ammonium group appears to offer a significant surface potential on the particle upon ionization. The charge that is developed at the surface of these nanospheres is believed to greatly enhance the stability of the suspension.

Thus, the EC/RS dispersion preferred in the present invention contains polymer particles i.e., nanospheres, which have an immobilized positive charge (quarternary ammonium groups in RS) on the surface. A resulting long-term stability is an advantage of the preferred invention system. Although cetyl alcohol and Tween 80 may not contribute too much to the stability of the final pseudolatex, they are required during preparations of the primary oil in water emulsion.

Plasticizers known in the art are also within the present invention. Since the invention including the preferred EC/RS dispersion is intended for use for coatings in controlled release pharmaceutical formulations, water-soluble plasticizers which may easily leach out in aqueous solvent may not be desirable. To evaluate plasticizers known in the art in the dispersion for such use, a free film is prepared. Triacetin is suggested by the art for use in free film preparations. Unfortunately, the film becomes brittle after soaking in water for a few hours. Therefore, various plasticizers are thus used to replace triacetin during free film preparations for film coating. Films made from Myvacet 9-45 available from Kodak are also waxy and brittle; however, the films become stronger after soaking in water for a few hours. Films made from Citroflex A-2, A-4, available from Pfizer and diethyl phthalate (DEP) are flexible and are not affected when soaked in water for five hours. However, after soaking in water overnight, these films also become somewhat brittle. The

poor solubility of these plasticizers is expected to maintain the integrity of the coatings during dissolution in aqueous medium.

Free films of different polymeric dispersions are prepared using triacetin (25%) as plasticizer. Softening temperatures of the films are determined by thermomechanical analysis (TMA). The softening temperature of EC/RS dispersion of the present invention is the average of those of EC and RS, while the softening temperature of the mixtures of the prior art is similar to those of RS. These latter results indicate that RS does not interact with EC in prior art mixtures thereof and the film of such mixtures softens as RS softens. In contrast, with the preferred EC/RS dispersion of the present invention as well as the other dispersions of the present invention, a stronger interaction between polymers, e.g., EC and RS at the molecular level appears to occur during the preparation described herein and the free film thereof shows new physicomechanical properties.

The coating process using the dispersion of the present invention is analogous to that known in the art.

During coating, it is found that the pellets agglomerate when the bed temperature is set at 35 °C or above for the preferred dispersion (EC-RS) of the present invention. Variations of coating conditions within the variables of the invention would be understood by an ordinarily skilled artisan. Any tackiness that is observed appears to be inherent with the acrylic-based polymer, when the bed temperature is raised (above 30 °C for RS). Preferably the inlet temperature is, therefore, adjusted to provide bed temperatures to avoid tackiness which for RS is, thus, approximately 20 °C. Adjusting this condition allows the pellets to fluidize effectively.

Drug release data is among the advantages for the present invention coating. Also, less agglomeration is found under the lower temperatures of the coating process now found to be useful in the invention. To reduce agglomeration further, 15% talc may be incorporated in the coating formulation.

Acrylic-based polymer, for example RS, content may be reduced from 50% to 33% in the polymeric dispersion of the present invention. As a result, a slower release profile is observed. Thus, reduction of acrylic-based polymer, for example RS, in the dispersion may contribute to a slower release rate. Therefore, drug permeability through the coating of the present invention, can be manipulated by varying the ratio of ethyl cellulose, such as EC, and polymethacrylate such as RS, in the present invention. A higher polymethacrylate (RS) content thus may provide more available permeation pathways for the solute to diffuse. Increasing the cellulose-derived polymer, such as EC, serves to reduce the diffusion rate, if desired.

In the following examples, low temperature may be used throughout the coating process, and complete film formation may not be achieved. Comparative dissolution studies of freshly coated pellets and pellets cured overnight at 55 °C show that a significant decrease in drug release rate may be achieved from cured pellets. Therefore, additional curing may be preferred.

Based on the above observations and dissolution data, the present invention cellulose-derived polymer and acrylic-based polymer (such as EC/RS) dispersion provides coatings in modified release formulations. Thus, granules prepared from the aqueous dispersion of the present invention may be used for a pharmaceutical.

It may be advantageous to mix various additions with the granules coated according to the invention. The additives include small amounts; for example, 0.5 to 1% by weight, of colloidal silicon dioxide, such as Aerosil®, which is known and is marketed by Degussa, Frankfurt (Fed. Rep. of Germany). The free flotability of the granules is improved by this addition. There can naturally be added to the coating-material mixture also other auxiliaries in small amounts, for example, dyestuffs or flavoring agents.

In summary, the pharmaceutically active granules according to the invention can be produced in a known manner. This applies also in the case of the coated granules. These are produced in the fluidized-bed spraying apparatus known for this purpose, or in coating pans. The dispersion of the present invention for coating is fed in as an aqueous dispersion at room temperature, and spraying is performed with air at a temperature of 25 to 35 °C. The individual granules are readily obtained in this manner, that is to say, no undesirable agglomeration of granules occurs.

The pharmaceutically active granules may be used according to the invention by pressing the granules together with a disintegrating agent having a high disintegrating capacity and good binding properties, and with the customary auxiliaries otherwise used for forming tablets, within a wide dosage range into molded shapes, for examples, tablets or capsule- or rod-shaped compressed products. The formed shapes thus obtained have the property of rapidly disintegrating into separate granules in the stomach of the person being treated and hence becoming well dispersed. A localized overconcentration of the active substance in the digestive tract is in this way prevented, and a uniform, slowly occurring release of active substance dispersed over a large resorption area is ensured. It may be verified by microscopic examination that the individual granules have scarcely been damaged as a result of compression, so that on release of the active substance from the granules, the active substance is able to bring into effect its original advantageous

properties virtually completed. When a formed or molded shape has to be produced with two or more active substances, the individually dyed pharmaceutically active granules can be prepared separately, a factor which facilitates identification, and which renders the patient aware of the fact that the preparation being taken contains two or more active substances.

Suitable as disintegrating agents having a high disintegrating capacity and good binding properties for the formed shapes obtainable according to the invention are in particular crosslinked polyvinylpolypyrrolidone (PVPP), for example Polyplasdone® XL marketed by the GAF Corporation, New York, NY (USA), or Kollidon® CL (BASF, Ludwigshafen/Rhein, Fed. Rep. of Germany).

Tablets are also the present invention. Such tablets may be prepared from coated granules or themselves coated.

In the case of the auxiliaries otherwise customarily used for tableting, these are in particular binders, lubricants, and antisticking agents.

The usual tablet-compressing machines can be used for producing the formed shapes obtainable according to the invention.

Since the mechanical strength of the formed shapes is surprisingly good, it is possible to produce all the desired customary forms, for example, tablets and capsules or rod-shaped molded products, with or without breaking grooves. These pressed products can be provided, if desired, with a protective coat of lacquer known for this purpose.

All pharmaceutical active substances which can be used for peroral administration and for which a delayed release in the gastrointestinal tract is desired are essentially suitable, in the form of granules or crystals of an appropriate size, for being processed according to the invention. The present invention is, however, particularly advantageous with respect to the use of active substances which, when used at a fairly high concentration, can cause local irritation of the mucous lining of the gastrointestinal tract, and which are administered in large single doses.

The present invention is further illustrated by way of the following example.

## EXAMPLE

### EC/RS Dispersion

I. Thirty grams of ethylcellulose (EC), thirty grams of Eudragit® RS100 (RS), noted above, and two grams of cetyl alcohol are dissolved in 333 ml of methylene chloride. One-half gram of Polysorbate 80, available as Tween® 80 from ICI America in Wilmington, Del., is dissolved in one liter of distilled water. The methylene chloride solution is dispersed slowly into the aqueous phase using a variable-speed stirrer[6] fitted with a three-blade stirring shaft. The crude emulsion is stirred under ambient conditions for 30 minutes, followed by ultrasonic agitation for five minutes. The emulsion is then homcgenized by passage through a microfluidizer operated at 6000 psi (42000000 Pascal). Three passes are used to obtain homogeneous emulsion with reduced droplet size. The organic solvent is removed from the emulsion by blowing air. The emulsion is continuously agitated at 42 °C throughout the process. Evaporation is continued until the volume of the product is reduced to 400 milliliters. The resultant pseudolatex contains approximately 15% weight per volume solids in water.

II. The procedure is also used to prepare a dispersion of a one-to-one ratio of ethylcellulose and Eudragit® RS.

## EMULSION STABILIZATION

Mixtures of surfactants composed of 0% to 100% of Tween 80 in Span 80[9] with 10% increments are prepared. Fifty milliliters each of the mixtures are added to 2 ml of distilled water in test tubes. Methylene chloride solution (0.7 ml each) containing EC and RS is added to each test tube and the mixture agitated ultrasonically. The degree of phase separation in the test tube is determined after one day.

## FREE FILM PREPARATIONS

A free film of EC/RS dispersion, prepared above in the Example, is prepared by spraying the aqueous dispersions on a rotating Teflon plate. The aqueous dispersions contain 25% triacetin as plasticizer based on polymer weight.

## THERMOMECHANICAL ANALYSIS (TMA)

A small sample (about 3 mm in diameter) is cut out of the free film and placed on a quartz sample tube in a thermomechanical analyzer. The sample space is purged with nitrogen gas and monitored at a heating rate of 10 °C/min. The softening temperature of the polymer is determined from the thermogram.

## COATING PROCEDURE

Coating formulations are prepared by mixing suspensions of talc in purified water with EC/RS dispersion and plasticizer and used to coat drug pellets. The coating suspensions are stirred throughout the coating process. The coating conditions are given in Table I.

TABLE I

| Processing Conditions for EC/RS Coated Drug Pellets (Strea I, Available From Aeromatic) | |
|---|---|
| Parameter | Setting |
| Inlet temperature | 24 °-26 °C |
| Outlet temperature | 20 °-22 °C |
| Fluidization air volume (fan) | 10-15 |
| Flow rate | 2-6 ml/min |
| Atomization air pressure | 0.4-0.8 bar (40000 - 80000 Pascal) |
| Nozzle width | 1.1 mm |

To reduce the tackiness of coated pellets, the inlet temperature is kept at very low temperatures during the coating process. Initially, about 2% hydroxypropylmethylcellulose is applied on the pellets to form a subcoat and avoid dissolution and migration of the pharmaceutically active substance, diphenhydramine HCl, during the early stages of coating. The coated pellets are divided into two portions. A portion is cured at 55 °C in an oven overnight; while the other portion is left at room temperature.

## DISSOLUTION

In vitro dissolution studies are carried out at 75 rpm using the USP Dissolution Apparatus II (paddle) method. The dissolution medium is purified water at 37 °C. Samples are withdrawn from the dissolution vessels at preset time intervals and an equivalent volume of fresh dissolution medium is replaced automatically. Assay of the released drug is conducted spectrophotometrically at 258 nm.

Results of dissolution studies are as follows in Tables II, III, IV, V, and VI.

TABLE II

| Drug Release Data of Coated Diphenhydramine HCl Pellets (Plasticizer = 20% Triacetin) | | |
|---|---|---|
| Time (hrs). | Percent Released | |
| | A* | B* |
| 0.25 | 23 | 15 |
| 0.5 | 78 | 59 |
| 1 | 94 | 89 |
| 2 | 97 | 96 |
| 4 | 99 | 100 |
| 6 | 100 | 100 |

A* not cured
B* cured at 55 °C overnight

TABLE III

| Drug Release Data of Coated Diphenhydramine HCl Pellets (Plasticizer = 25% Citroflex A-2) | | |
|---|---|---|
| Time (hrs). | Percent Released | |
| | A* | B* |
| 0.16 | 5 | 5 |
| 0.33 | 48 | 38 |
| 0.5 | 90 | 81 |
| 1 | 99 | 93 |
| 1.5 | 99 | 94 |
| 2 | 99 | 94 |

A* not cured
B* cured at 55 °C overnight

TABLE IV

| Drug Release Data of Coated Diphenhydramine HCl Pellets (Plasticizer = 20% DEP) | | |
|---|---|---|
| Time (hrs). | Percent Released | |
| | A* | B* |
| 0.16 | 10 | 8 |
| 0.33 | 20 | 15 |
| 0.5 | 29 | 21 |
| 1 | 65 | 45 |
| 1.5 | 89 | 74 |
| 2 | 92 | 86 |

A* not cured
B* cured at 55 °C overnight

TABLE V

| Drug Release Data of Coated Diphenhydramine HCl Pellets (Plasticizer = 25% DEP, 15% Talc Added) | | |
|---|---|---|
| Time (hrs). | Percent Released | |
| | A* | B* |
| 0.25 | 2 | 1 |
| 0.5 | 3 | 1 |
| 1 | 7 | 3 |
| 1.5 | 17 | 12 |
| 2 | 50 | 33 |
| 3 | 92 | 75 |
| 4 | 97 | 89 |
| 6 | 100 | 96 |

A* not cured
B* cured at 55 °C overnight

TABLE VI

| Drug Release Data of Coated Diphenhydramine HCl Pellets (EC/RS = 2/1, Plasticizer = 25% DEP, 15% Talc Added) | | |
|---|---|---|
| Time (hrs). | Percent Released | |
| | A* | B* |
| 0.25 | 4 | 3 |
| 0.5 | 6 | 4 |
| 1 | 11 | 7 |
| 2 | 37 | 21 |
| 4 | 87 | 66 |
| 6 | 93 | 92 |
| 8 | 96 | 96 |

A* not cured
B* cured at 55 ° C overnight

Other pharmaceutically active substances, such as known to one of skill in the art, or the like, may be used to prepare pellets according to the above example.

## Claims

1. A process for the preparation of a dispersion which comprises 1) mixing a cellulose-derived polymer and an acrylic-based polymer in an organic solvent, 2) emulsifying the mixture of step 1) in water, and 3) removing the organic solvent from the emulsion.

2. A process of Claim 1 wherein the organic solvent is methylene chloride.

3. A process of Claim 2 wherein the cellulose-derived polymer is ethyl cellulose, cellulose acetate or acetate phthalate, and the acrylic-based polymer is polymethacrylate.

4. A process of claims wherein the polymethacrylate is Eudragit® RS or RL.

5. A process of Claim 1 wherein the ratio of the cellulose-derived polymer and the acrylic-based polymer is of from 1:3 to 3:1.

6. A process of Claim 5 wherein the ratio is of from 1:1 to 2:1.

7. The dispersion which is prepared according to the process of Claim 1.

8. A composition which is prepared by removing the water from the dispersion of Claim 7 to form a pseudolatex having 15 to 30% weight per volume solids in water.

9. A composition consisting of particles consisting essentially of a cellulose-derived polymer and an acrylic-based polymer.

10. A pharmaceutical dosage form having a coating consisting essentially of the composition of Claim 9.

11. The dosage form of Claim 10 which is in the form of granules.

12. The dosage form which is a tablet, capsule, or suspension having the granules of Claim 11.

13. The dosage form of Claim 10 consisting of a tablet.

14. The process of preparing a pharmaceutical dosage form using the dispersion prepared according to claims 1 to 6 for coating a solid pharmaceutical preparation containing at least one pharmaceutically active compound and usual adjuvants.